# EUROPEAN PATENT SPECIFICATION

(11) **EP 2 416 696 B1**
(45) Date of publication and mention of the grant of the patent: **11.06.2014**
(21) Application number: 10720360.6
(22) Date of filing: 06.04.2010
(51) Int. Cl.: A61B 3/032

(54) **OPTOMETRIC TESTING SYSTEM**
OPTOMETRISCHES TESTSYSTEM
SYSTÈME DE TEST OPTOMÉTRIQUE

(30) Priority: 06.04.2009 GB 0905957
(43) Date of publication of application: 15.02.2012
(73) Proprietor: City University, London EC1V OHB (GB)
(72) Inventor: ZHU, Haogang, London EC1V 0HB (GB); SMITH, Nicholas, London EC1V 0HB (GB); CRABB, David, London EC1V 0HB (GB); GRUPPETTA, Stephen, London EC1V 0HB (GB)
(74) Representative: Milhench, Mark Lorne
(86) International application number: PCT/EP2010/054505
(87) International publication number: WO 2010/115870

(56) References cited:
- WO-A1-00/13572
- WO-A2-2007/026368
- US-A- 5 420 652

## Description

### Field

This invention relates to optometric testing systems. One particularly preferred illustrative embodiment of the present invention relates to a portable optometric testing system.

### Background

It is unfortunately the case that whilst the provision of high quality public health care is a laudable aim, the actuality is that the delivery of such a service is often hampered by limited resources, such as a lack of appropriate equipment (typically because it is too expensive) and too few suitably qualified clinicians. As a result of this, access to timely healthcare for much of the population is hampered, and as a consequence the awareness of diseases or conditions that may impact on quality of life is delayed.

This scenario is especially apparent in the field of eye care. Loss of vision can arise from many conditions, of which glaucoma, age-related macular degeneration and diabetic retinopathy are illustrative examples. Whilst vision loss due to such conditions is often ultimately irreversible, it is nevertheless the case that before a loss of vision becomes severe and irreversible, functional vision testing (for example: colour testing, motion detection testing or contrast sensitivity testing) could be employed to identify the onset of such conditions at a time where treatment is still a clinical possibility.

In the particular case of glaucoma (an age-related chronic disease that adversely affects visual function and which can lead to irreversible blindness if untreated) it has been suggested in a report entitled "The cost of blindness" (prepared for the Guide Dogs for the Blind Association by C. Langley-Hawthorne and published in 2003) that in the United Kingdom a saving of up to £1 Billion could be made if just 10% of glaucoma patients had their conditions detected sooner and received earlier treatment to arrest the disease process. In addition, an article in the British Medical Journal entitled "Preventing Blindness from Glaucoma" by D.B. Henson and R. Thampy (BMJ 331: 120-1, 2005) suggested that an imperative for better glaucoma detection is simply increased access to and availability of appropriate testing.

On this basis, it would be advantageous - both in financial terms and health terms - if patients could be provided with ready access to comprehensive eye health testing facilities. The reality is, however, that whilst some patients may have access to acceptable or indeed superlative levels of care, it is difficult to provide the population as a whole with a reasonable level of care.

One reason for this is that conventional visual function test systems (for example the Humphrey® Visual Field Analyser manufactured by Carl Zeiss Meditec Vertriebsgesellschaft mbH, Göschwitzer Str. 51-52, 07745 Jena, Germany) are relatively expensive, single-purpose devices and as such many optometrists do not have a full range of machinery available to them.

Another reason is that as it can sometimes be difficult to get an appointment with an optometrist or clinic; patients tend not to go for testing until their visual function has already degraded to a point where there is a noticeable defect or loss of vision. However, for progressive retinopathy such as glaucoma, when a patient begins to notice a defect in their field of view it is typically the case that irreversible damage has already occurred. This is a particular problem for the elderly, not only because as they are older they are more likely to suffer from vision problems, but also because such patients can often be house bound or living in care homes and hence may not easily be able to attend an eye clinic.

Yet another problem is that as eye testing is typically extremely sensitive to environmental and operator variations (principally due to the relative inflexibility of the instruments and the lack of a control), problems can be difficult to diagnose and follow up. As a consequence of this, vision function tests undertaken independently tend to show little concordance, and as such it is often necessary - to achieve a reliable diagnosis - for multiple tests to be undertaken, thereby further consuming limited resources.

Another related problem is that as visual function testing systems tend to be single-purpose devices, it is difficult if not impossible to correlate results obtained from different machines.

Whilst these difficulties affect a significant proportion of the UK population, in poorer countries it is not unusual for up to ninety percent of the population to experience serious problems obtaining adequate care.

It is apparent, therefore, that it would be advantageous to provide an optometric testing system that avoids or at least mitigates these problems.

Additional prior art includes WO2007/026368, US5420652 & WO00/13572.

WO2007/026368, on which document the preamble of claim 1 is based, relates to a multi-functional optometric - ophthalmic system for testing, diagnosing, or treating, vision or eyes of a subject, and methodologies thereof. The system includes: a head mountable unit, including a head mounting assembly, and at least one near eye module assembly, mounted upon the head mounting assembly, for generating optical processes or effects which act or take place upon, and are affected by, at least one eye of the subject, and for receiving results of the optical processes or effects from the at least one eye, as part of the testing, diagnosing, or treating of the vision or eyes of the subject; and a central controlling and processing unit. Near eye module assembly includes: a micro-display, a first lens assembly, and a refraction correction assembly. Generally applicable for performing a wide variety of different optometric and ophthalmic tests, diagnoses, and treatments, of a subject's vision or eyes.

US5420652 relates to a visual acuity test mark displaying device that comprises: a test mark selecting means provided with a plurality of test mark selecting keys which are operated for the sequential selection of visual acuity test marks; a display means for displaying visual acuity test marks selected by the test mark selecting means at a predetermined position; a specifying means for specifying optional key-mark combinations each of a test mark selecting key and a visual acuity test mark selected by operating the same test mark selecting key; an input means for entering data representing the arrangement of the visual acuity test marks specified by the specifying means; and a storage means for storing the optional key-mark combinations specified by the specifying means. The visual acuity test mark displaying device can be set for visual acuity test mark sets desired by the user and enables the change of the visual acuity test mark sets for which the same is set beforehand.

WO00/13572 relates to a multi-functional, visual test instrument which is realized by integrating miniature, close proximity displays, such as LCD, with viewing optics, which is constructed in the form of preferably a unitary housing. In a preferred embodiment, the multi-functional, visual test instrument includes two viewing assemblies, and displays, which are all enclosed in the housing that is slidable or pivotally attached to a movable mount, such as a slit lamp base. In operation, various optical test objects or stimuli are viewed by the patient on the displays through the viewing assemblies. The shape, size, speed, frequency, location, color, contrast and intensity of the test objects or stimuli are computer generated. A hand operated switch, mouse, joystick, or other input means, may be used for the patient to respond to the observed test objects or stimuli.

### Summary

According to an aspect of the present invention there is provided an optometric testing system as defined in Claim 1.

Preferred features and advantages of this and other envisaged arrangements are set out elsewhere in the accompanying claims and following detailed description.

### Brief Description

Various aspects of the teachings of the present invention, and arrangements embodying those teachings, will hereafter be described by way of illustrative example with reference to the accompanying drawings, in which:
Fig. 1 is a schematic representation of an optometric testing system according to a preferred embodiment of the present invention;
Fig. 2 is a schematic view in perspective of optics for use with the system of Fig. 1;
Fig. 3 is a schematic view of the optics of Fig. 2 in a wide-field configuration;
Fig. 4 is a schematic view of the optics of Fig. 2 in a narrow-field configuration;
Fig. 5 is a schematic representation of the software functionality provided by the system; and
Fig. 6 is a schematic representation of an optometric testing system according to another embodiment of the present invention.

### Detailed Description

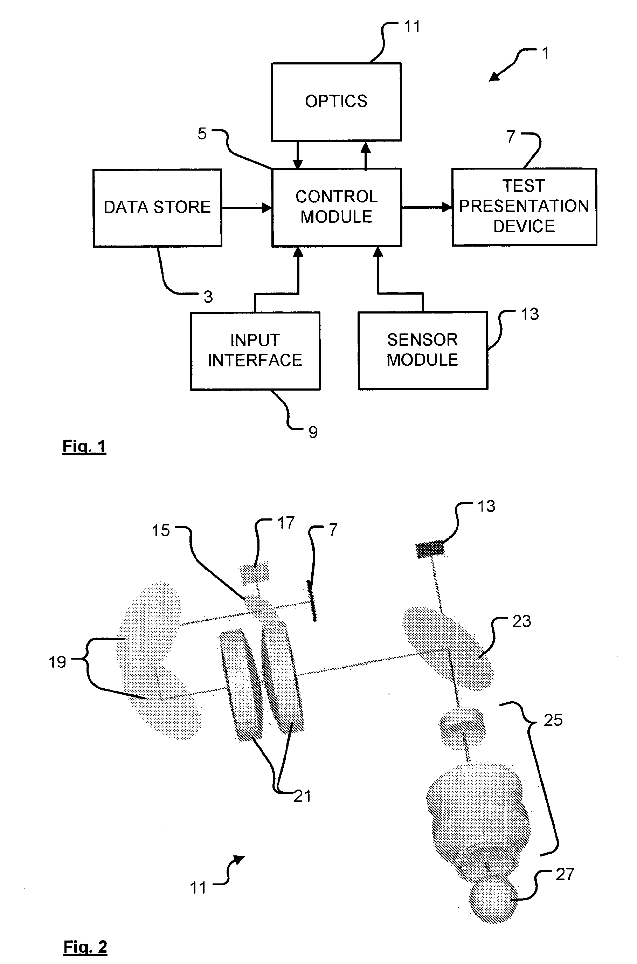

Referring now to Fig. 1 of the accompanying drawings, there is depicted a schematic representation of an optometric testing system 1 according to a first envisaged implementation. In general terms, this first arrangement is particularly well suited for use as a portable testing system.

The system 1 of this arrangement comprises a data store 3 in which a library of different optometric and ophthalmic tests is provided. Each test is stored in the form of a set of instructions that, when executed by a processor, control a test presentation device to provide optical stimuli for viewing by a subject. In envisaged arrangements the system is designed for comprehensive visual function testing and the data store includes instructions for a suite of different tests, for example one or more of a light sensitivity test, a contrast sensitivity test, a visual field test, a motion detection test (for example, the Moorfields Motion Detection Test (see: www.moorfieldsmdt.co.uk for details)), distance charts, and a colour vision test (in particular a test for age related macular degeneration), as well as any of a number of other tests known to persons of ordinary skill in the art.

As will be appreciated, the more comprehensive the list of tests stored in the data store, the closer the optometric system is to functioning as a comprehensive 'one-stop' visual function testing system. From the clinician's point of view, such an arrangement is highly advantageous because the clinician no longer needs access to a plurality of different machines to obtain a better appreciation of the condition of the subject's eyes. Furthermore, as the system of this arrangement employs the same equipment for a variety of different tests, irregularities previously associated with the different displays, for example, of a number of single-purpose machines can be avoided.

The data store 3 can be implemented by means of any device or series of devices that is capable of storing data. In one envisaged arrangement the data store comprises one or more hard disk data storage devices. In another arrangement the data store could comprise a solid state disk (SSD), memory or a remote repository accessible via a data communications network (for example an internet such as the world wide web).

The data store is coupled to a control module 5 that includes a processor and memory (neither of which are shown). The processor is configured to execute instructions embodied in visual tests retrieved from the data store 3, and to control a test presentation device 7 in accordance with those instructions to provide visual stimuli for viewing by a subject. The control module may comprise, for example, a computing resource (such as a personal computer) or application specific hardware (for example an application specific integrated circuit (ASIC)). In one particularly preferred arrangement the data store and control module comprise respective parts of one personal computer, for example a portable or so-called laptop computer.

The test presentation device 7 comprises, in general terms, any device that is capable of providing optical stimuli for viewing by a subject. In one illustrative example the test presentation device 7 comprises a high quality, high contrast display, for example an SXGA (Super eXtended Graphics Array) compatible display with a resolution of 1280 x 1024 pixels. For example, the test presentation device may comprise a reflective micro-display that has a diagonal screen size that is in the order of a few centimetres, for example less than roughly 2 cm. In one implementation the display may comprise a so-called liquid crystal on silicon (LCoS) display that can generate a high-contrast and high-resolution SXGA image. In another implementation example the test presentation device may comprise an OLED (organic light emitting diode) display that provides a comparable resolution to that of the SXGA display.

As will be appreciated by persons skilled in the art the use of a single test presentation device for a number of different tests helps avoid problems associated with correlating results obtained from different items of equipment that each has its' own test presentation device.

An input interface 9 is coupled to the control module 5, and is used by a subject in the course of a given test to respond to stimuli provided to the subject by means of the test presentation device 7. The input interface may comprise one or more switches, buttons or any other means that that can be operated by a subject to generate a signal that informs the processor 5 of the control module that a given stimulus has been viewed.

By way of illustration, in a visual field test one of the subject's eyes is covered and they are instructed to look at a point at the centre of the test presentation device. To test the subject's visual field illuminated points are presented and the subject is instructed to operate the input interface 9 on each occasion that a spot of light is seen. The processor of the control module 5 collates a list of points that the subject has correctly identified. In one particular example, the examination tests fifty-two points in the central 24° field. These points are distributed at eccentricities of 3°, 9°, 15°, 21° and 27 ° in the nasal region. The points are strategically located so as to increase the chances of detecting glaucomatous nasal steps and arcuate defects, hemianopia and quadrantanopia caused by brain diseases, and paracentral defects attributable to macular degeneration and drug toxicities.

In this arrangement, the control module 5 is coupled to optics 11 that comprise (as will later be described in detail) a series of optical components that are arranged to permit variable narrow and wide angle fields of view of the test presentation device 7 and an adjustable focus.

In contrast to a typical virtual reality (VR) type display system, the optics 11 can-in one particularly preferred arrangement - be configured by the control module 5 to provide angles of up to 80° for monocular field of view; whereas VR systems typically only provide 30° to 40° of monocular field of view. As mentioned above, the test presentation system 7 provides visual stimuli for viewing by subjects being tested, and in general terms the optics 11 function to relay the image from the test presentation device 7 onto the subject's retina with the appropriate focus, field of view and at infinite conjugate. The field of view in particular is variable to accommodate the different needs of the various tests implemented.

In one particularly preferred arrangement the distance from the eye to a virtual image projected by the optics 11 is adjustable according to the spectacle prescription (if any) of the subject, and a principal advantage of this arrangement is that a subject undergoing testing with the system of the present invention can more easily participate without vision correction than with dedicated testing equipment of the type previously proposed.

As shown in Fig. 1, in this arrangement the control module 5 is also coupled to a sensor module 13. In this arrangement the sensor module 13 functions as a sight line sensor (i.e. as a means for determining the subject's line of sight to the test presentation device) and to this end includes an eye tracker which is configured to monitor the eye of a subject and to determine which point of the test presentation device the subject is looking at in the course of a test. The eye tracker allows the system as a whole to implement automatic quality control so that the quality of collected data can be improved. For example, in the context of a visual field test of the type aforementioned it is important for a subject to look straight ahead at a fixation point provided on the test presentation device 7, and if the subject should fail to do this the accuracy of the results of the test may be compromised.

This arrangement differs from conventional systems where it is usual for a chin rest and/or head restraints to be employed so that movement of a subject's head relative to the test presentation device is resisted, and as will be appreciated such conventional systems cannot account for errors attributable to a subject who fails to look at the straight-ahead fixation point.

By employing a sensor module of the type aforementioned the system of this arrangement can monitor eye movements of the subject and the control module can, in response to a detected diversion from the fixation point, adjust the position at which a given visual stimuli of a selected test is provided so that the stimulus in question is correctly positioned (for viewing by a particular part of a the subject's eye) with respect to the direction in which the subject is currently looking, rather than being positioned with reference to a fixation point that the subject may not actually be looking at. As will be appreciated, by virtue of this arrangement the accuracy of the testing can greatly be improved.

The eye tracker of the type aforementioned can also be employed to provide the subject with the ability to interact with the control module, for example to control the system. In one illustrative example the system could be configured to control the test presentation device to provide a virtual button and to determine that a "press" of that button has occurred when a user has looked at the virtual button for more than a predetermined period of time.

In one envisaged implementation the eye tracker comprises a CCD sensor or other image capture device which is capable of capturing an image of the subject's eye for further processing, for example by the processor of the control module 5. The processor may, in one arrangement, be configured to invoke a software module which detects, for example by means of a robust ellipse fitting algorithm (the like of which is well known in the art), the edge of the pupil in the image of the subject's eye.

As will be appreciated, the shape of the ellipse fitted to the pupil in the image of the subject's eye will change as the subject's eye moves, and the processor of the control module (for example) can determine, in real time, from the shape of the ellipse the direction in which the subject's eye is looking and hence the part of the test presentation device that the eye is looking at. The processor of the control module 5 (for example) can then alter the position at which a given stimulus is to be provided in accordance with the determined sight line of the subject.

The sensor module may also include one or more other sensors, for example a light intensity sensor, that are capable of monitoring the ambient environment, for example to determine the ambient light level for the environment in which the system is being operated. The control module may then control the test presentation device 7, for example to adjust the contrast and/or brightness of images provided thereby, to account for variations in ambient light levels throughout the test, or indeed between ambient levels on different occasions when the test has been taken. For example a subject may undertake a test at a given point in time, and then be retested several months later. If the tests are taken outside of a controlled clinical environment, and if the first test is taken during the summer then it may well be the case that a greater amount of ambient light levels may be attributable to natural sunlight than would be the case if the subsequent test is taken during the winter when a larger proportion of the ambient light may be attributable to artificial light sources. By providing a sensor module that incorporates a light intensity sensor it is possible to adjust the contrast and/or brightness of the testing images to be consistent, for example throughout any one test or between an original and a repeated test.

In this first envisaged arrangement the system may be configured to be readily portable so that it can be taken to subjects, thereby relieving subjects from having to travel to have their eyes tested. For example, at least the optics 11, the test presentation device 7 and the sensor module (if provided) may be configured to be components of a device that a subject wears on their head (in a manner similar to a helmet) and which interfaces with a portable computing resource, such as a laptop computer, which provides the control module 5, data store 3 and input interface 9. In another envisaged implementation, the data store and control module may comprise components of a wearable computing resource that is built into the head-mounted device. This latter arrangement provides a particularly compact arrangement that is readily portable.

Referring now to Fig. 2, there is depicted a schematic perspective view of the optics 11 of the system 1. As will be appreciated by persons skilled in the art, whilst the arrangement shown in Fig. 2 is preferred on account of the fact that it is relatively compact and hence easily portable, the arrangement of the optical components may be varied without losing the functionality provided by the optics and as such the particular arrangement shown in Fig. 2 should be considered as being merely illustrative of the teachings of the present invention.

The optics 11 includes a polarising beamsplitter 15 that is configured to reflect polarized light from a light source 17 onto the display, and to pass reflected light with orthogonal polarisation from the display to a pair of mirrors 19. Light reflected from the mirrors 19 passes through relay optics 21 to a dichroic mirror 23, and is reflected by the dichroic mirror 23 to an eyepiece 25 that is configured to form an image on the retina of the subject's eye 27. The dichroic mirror 23 is configured to reflect light from light source 17 and to transmit light of a particular range of wavelengths reflected back from the subject's eye.

A light source (not shown) that is capable of producing light of a wavelength that is transmitted by the dichroic mirror 23 is configured to illuminate the subject's eye, and light reflected from the eye of the subject passes back through the eyepiece 25 and through the second dichroic mirror 23 to an image capture component 13, for example a CCD chip, of the aforementioned eye tracker. In one envisaged arrangement the dichroic mirror 23 is configured to transmit infra-red light and the light source for illuminating the subject's eye comprises a infra-red light source, such as a plurality of infra-red LEDs.

In one envisaged implementation the relay optics 21 may comprise two achromat lenses, and the eyepiece 25 may comprise a telescope eyepiece (i.e. an eyepiece providing a wide field of view) such as a Nagler eyepiece.

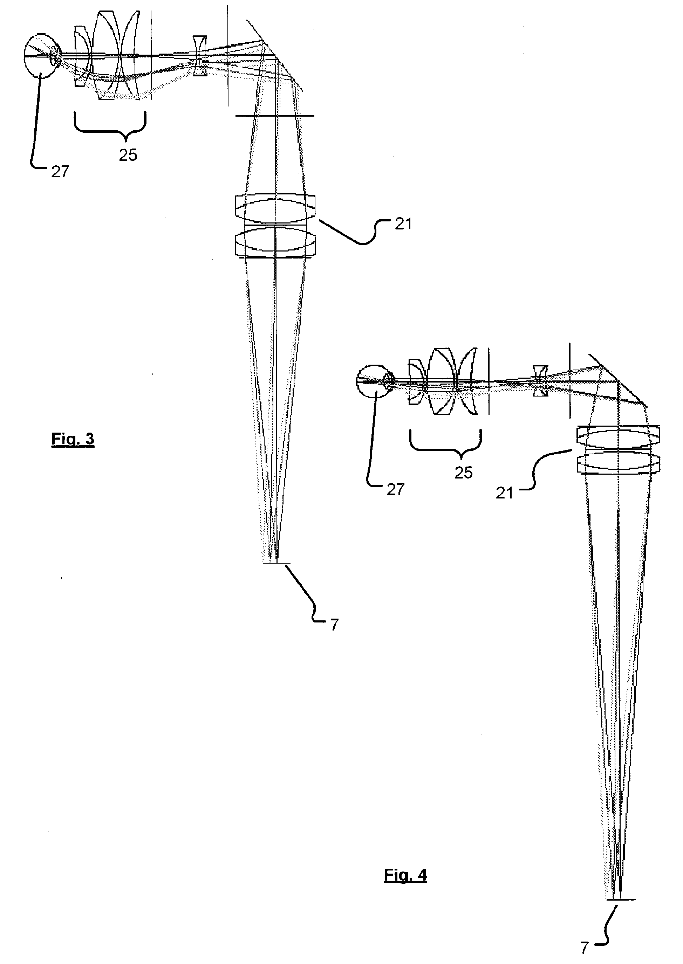

The optics are coupled to a drive mechanism (not shown), such as a worm drive for example, that is controlled by the control module and can be accurately moved to vary the distances between the relay optics 21, the test presentation device 7, and the eyepiece 25. As will be appreciated by persons skilled in the art, this arrangement provides - in a particularly compact form - an optical assembly that provides an adjustable and wide field of view that is well suited for visual function testing purposes. In particular, by moving the relay optics 21 towards the test presentation device 7, the field of view increases and by moving the relay optics 21 away from the test presentation device the field of view narrows. For example as shown in Figs. 3 and 4, the field of view for the arrangement depicted in Fig. 3 is in the region of 80°, whereas the field of view for the arrangement depicted in Fig. 4 (with the relay optics further from the test presentation device) is narrower.

As aforementioned, the testing system 1 is controlled by a control module 5 that includes a processor which is configured to establish an operating system and execute software instructions to provide visual function tests, log subject responses, control operation of the system and - optionally - provide automated diagnosis of potential problems.

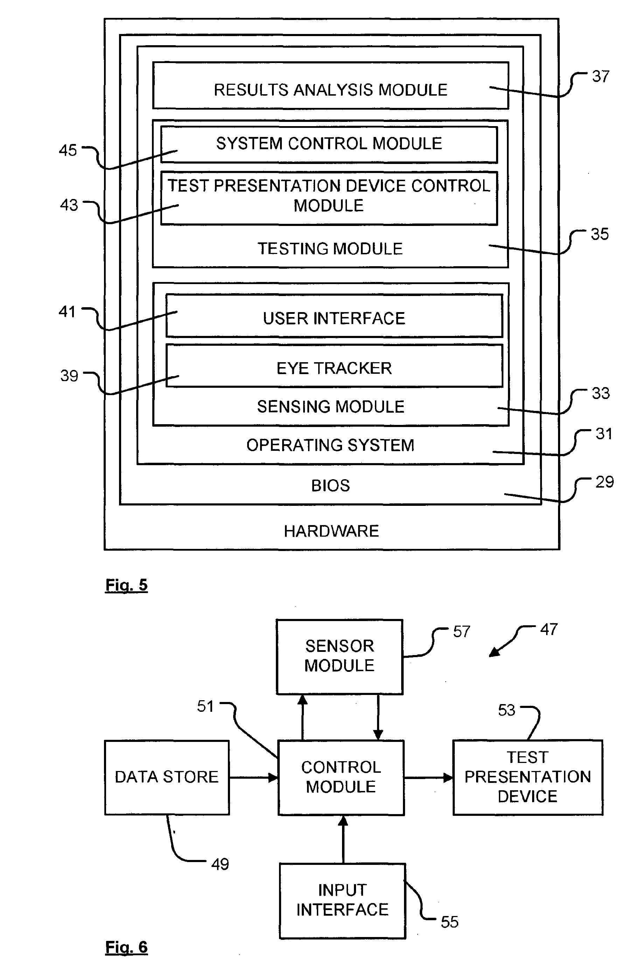

Referring now to Fig. 5, there is depicted a schematic representation of the various software modules that are invoked and executed by the processor of the control module 5. The processor of the control module 5 cooperates with memory (not shown) to establish a BIOS (Basic Input/Output System) 29 that functions as an interface between the functional hardware components of the system 1 and the software executed by the device. The processor then loads, for example from memory, an operating system 31 which provides an environment in which application software (implementing some or all of the above described functionality) can run. In accordance with a preferred implementation, the application software includes a sensing module 33, a testing module 35 and a results analysis module 37.

The results analysis module may comprise part of the application software of the testing system, or in another envisaged arrangement the testing system could output the results of any testing to a separate processing system that is configured to invoke and execute the software functionality of the results analysis module.

The sensing module includes sight line sensor module, in this instance an eye tracker module 39 that processes images captured by the image capture component 13 and invokes the aforementioned ellipse matching algorithm to locate the pupil and from the shape of the ellipse determine the location of the test presentation deivce that the subject is looking at. The sensing module also includes a user interface 41 which interprets signals received from the user interface hardware such as a keyboard, button or mouse and interprets those signals, depending on the mode in which the system is operating, as user input in response to optical stimuli or user system control selections. User inputs in response to optical stimuli of a given test are logged as results for that test, and stored for example in the data store 3 or in memory. Optionally, as aforementioned, the user interface may include software functionality that controls the test presentation device to provide images of virtual buttons that a user can select by gazing at a given button, and functionality responsive to any other sensors, for example an intensity sensor of the type aforementioned.

The testing module 35 comprises a test presentation device control module 43 and a system control module 45. The testing module 35 is capable of interfacing with the data store 3 to retrieve stored eye tests, and the test presentation device control module 43 interprets the instructions embodied within a selected eye test and controls the test presentation device 7 to provide stimuli at appropriate locations for the selected test. The test presentation device control module also cooperates with the system control module, as required for a test being undertaken, to adjust the optics 11 and thereby vary the field of view.

The results analysis module, which in this illustrative arrangement comprises part of the testing system 1, is configured following completion of a given test to retrieve the logged results from that test and invoke known analytical techniques to provide an indication as to whether the subject undertaking the test is exhibiting a loss or degradation in vision.

In addition, in a departure from conventional testing equipment, because the system of this arrangement can use a homogenous set-up for multiple tests, the results of a number of different tests may validly be analysed to provide a more comprehensive diagnosis. As described in "Structure and Function in Glaucoma: The Relationship between a Functional Visual Field Map and an Anatomic Retinal Map" by Strouthidis, N.G., Vinciotti, V., Tucker, A.J., Gardiner, S.K., Crabb, D.P., and Garway-Heath, D.F. ((2006) Invest Ophthalmol Vis Sci 47: 5356-5362) an expert system, driven by artificial intelligence methods, can be configured to work on the results from various tests, and automatically identify apparent defects in visual function.

Diagnosis results can be output for consumption by the subject taking the tests or a medical practitioner, and in a particularly preferred arrangement stored tests can be personalised (in an appropriate manner for any diagnosed defects) for the subject and stored for retrieval at a later date when the subject is retested. For example, if a first round of testing should suggest that a subject has a defect with a particular part of their eye, the testing may be personalised to concentrate on that apparent defect in future testing sessions. By virtue of this arrangement clinicians are provided with more detailed information on which to base their decision-making.

In a modification of this first envisaged arrangement, the test presentation device may comprise a scanning imaging device that implements a raster scan of a beam of light over the retina of a subject in a manner akin to that of a Scanning Laser Ophthalmoscope (such as the HRT instrument provided by Heidelberg Engineering). In an envisaged implementation for provision of a colour image for viewing by a subject, three coincident light beams (one red, one green and one blue) from respective sources (such as laser light sources) are deflected horizontally and vertically, for example by scanning mirrors, to produce a rectangular raster. In a preferred arrangement, the horizontal scan is typically over 160 times faster than the vertical scan (for a video rate display) so that the rectangular raster is made up of a series of horizontal lines. Optics are then used to focus the rectangular raster onto the subject's retina. The beams are individually intensity modulated in synchronisation with the devices (for example scanning mirrors) used to horizontally and vertically deflect the beams so that the rectangular raster is divided into pixels (the number of which depends on the source modulation speed). The relative intensities of the three sources at any one point in time enable each pixel to take any of a series of colours within the visible spectrum, and over the entire raster the subject whose retina is being scanned perceives a colour image. In another envisaged implementation for provision of monochromatic images to a subject, a single white light source (for example a laser light source) may be provided and raster scanned over the subject's retina.

In a second envisaged arrangement that is of particular use in clinical environments (such as a hospital) where cross-infection between subjects might be of concern and hence a helmet style implementation may be less preferred, the system may be configured as shown in Fig. 6. In this arrangement of the invention the system as a whole will likely be less portable than the system of the first arrangement, and intended for installation and operation in one location. That is not to say that this arrangement of the invention cannot be moved from one location to another, merely that as it will normally be used in one location it is not configured to be readily portable.

In this envisaged implementation the system 47 comprises a data store 49 that can again be implemented by means of any device or series of devices that is capable of storing data. As before, in one envisaged arrangement the data store comprises one or more hard disk data storage devices, a solid state disk (SSD), memory or a remote repository accessible via a data communications network (for example an internet such as the world wide web).

The data store 49 is coupled to a control module 51 that includes a processor and memory (neither of which are shown). The processor is configured to execute instructions embodied in visual tests retrieved from the data store 49, and to control a test presentation device 53 in accordance with those instructions to provide visual stimuli for viewing by a subject. In this arrangement it is envisaged that the control module will comprise, for example, a computing resource such as a desktop personal computer.

The control module 51 is coupled to a test presentation device 53 that comprises a high quality, high contrast display, for example an SXGA (Super eXtended Graphics Array) compatible display with a resolution of at least 1280 x 1024 pixels. As will be appreciated by persons skilled in the art a variety of computer monitors are available that provide the requisite quality and resolution, and any of these monitors may in principle be employed in this arrangement.

An input interface 9 is coupled to the control module 5, and is used by a subject in the course of a given test to respond to stimuli displayed on the test presentation device 53. The input interface may comprise one or more switches, buttons or any other means that that can be operated by a subject to generate a signal that informs the processor of the control module 51 that a given stimulus has been viewed.

The control module 51 of this envisaged implementation is coupled to a sensor module 57 that includes a light intensity sensor that is capable of monitoring the ambient environment to determine the ambient light level for the environment in which the system is being operated. As with the system of the first arrangement, the control module 51 may then control the test presentation device 53, for example to adjust the contrast and/or brightness of images provided thereby, to account for variations in ambient light levels throughout a test, or indeed between ambient levels on different occasions when a test has been taken.

The sensor module 57 of this arrangement may also include a sight line sensor, and in this arrangement the sight line sensor is configured to determine the position of the subject's head relative to the test presentation device and infer from that determination an indication of the region of the test presentation device that the subject is looking at. In one proposed implementation, the sight line sensor may comprise an image capture device whose position relative to the test presentation device is known. The image capture device, which may comprise a camera, is configured to capture an image of a target whose position is fixed relative to a subject. The target may comprise a marker applied to a subject (for example an annular sticker fixed to the subject so that it lies around the eye being tested) or a marker carried by a device (for example an empty spectacle frame) so as to at least partly surround the eye being tested.

The image capture device is coupled to the processor of the control module 51 (for example), and in a preferred arrangement the processor is configured to process images from the image capture device to locate the marker and determine the degree (if any) to which the marker has moved from a previously determined position where the subject is known to be looking straight ahead at the test presentation device. In a similar manner to the arrangement described in connection with the first arrangement, in a preferred implementation the control module 51 may be configured to adapt the presentation of a test to a subject, in real time, depending upon the detected position of the subject's head relative to the test presentation device.

Detection of the target may be accomplished, as before, by invoking pattern matching algorithms to initially locate the target and subsequently by matching ellipses to the

In another contemplated arrangement, the sight line sensor may be omitted from the sensor module 57, and a conventional chin rest and/or head restraint be provided to stabilise the head of the subject with respect to the test presentation device.

As with the system of the first arrangement, the testing system 47 of this implementation is controlled by a processor of the control module 51 that is configured to establish an operating system and execute software instructions to provide visual function tests, log subject responses, control operation of the system and - optionally-provide automated diagnosis of potential problems.

The software modules employed for this purpose are generally the same as those of the first arrangement, and for brevity the functionality of common components will not be repeated.

The chief difference between the software modules of this arrangement and that of the first arrangement, is that in this arrangement the sensing module need not include a sight line sensor module, and that if such a module is provided it functions to control the image capture device to capture images of the target and to control a processor to interpret those images (for example using a robust ellipse fitting algorithm of the type aforementioned) to locate the target in the image and derive therefrom an indication of the position of the subject's head with respect to the test presentation device.

It will be apparent from the foregoing that the implementations described herein provide an advantageous arrangement as compared with the dedicated stand-alone equipment that characterises the prior art. It will also be appreciated that whilst various aspects and implementations have heretofore been described, the scope of the present invention is not limited to the particular arrangements set out herein and instead extends to encompass all arrangements, and modifications and alterations thereto, which fall within the scope of the appended claims.

## Claims

1. An optometric testing system (1, 47) comprising:
a test presentation device (7, 53) for presenting optical tests to a subject;
a data store (3, 49) for a library of different optometric tests, each said test comprising instructions for providing optical stimuli to a subject;
a control module (5, 51) responsive to the instructions stored in said data store (3, 49) for a selected test to control the test presentation device (7, 53) to provide the stimuli for that test to the subject; and
an input interface (9, 55) operable by the subject to generate signals in response to the stimuli of a selected test that are presented to said subject; said control module (5, 51) being configured to receive said signals from said input interface (9, 55) in response to the stimuli of said selected test;
wherein said optometric testing system (1, 47) further comprises a sensor module (13, 57), said control module (5, 51) being configured to control said test presentation device (7, 53) in accordance with signals received from said sensor module (13, 51), **characterised in that** said sensor module (13, 57) includes a line of sight sensor and that said control module (5, 51) is configured to adjust a test strategy of a selected test in response to signals received from said line of sight sensor.

2. A system (1, 47) according to Claim 1, wherein said control module (5, 51)is configured to adjust a test strategy of a selected test by varying display locations for stimuli.

3. A system (1, 47) according to Claim 1, wherein said line of sight sensor and said control module (5, 51) cooperate to track movement of an eye of a subject.

4. A system (1, 47) according to Claim 1, wherein said line of sight sensor and said control module (5, 51) cooperate to track movement of a subject's head.

5. A system (1, 47) according to any of claims 1 to 4, wherein said sensor module (13, 57) comprises one or more environmental sensors.

6. A system (1, 47) according to Claim 5, wherein said sensor module (13, 57) comprises a light intensity sensor.

7. A system (1, 47) according to any preceding claim, wherein said test presentation device (7, 53) comprises a display.

8. A system (1, 47) according to any of Claims 5 to 7 when dependent on any of claims 1 to 3, wherein said display comprises a micro-display and said system further comprises optics (11) for projecting images displayed on said display onto the retina of a subject being tested.

9. A system (1, 47) according to Claim 8, wherein at least said display and said optics (11) are provided as components of a head mountable device.

10. A system (1, 47) according to Claim 9, wherein said head mountable device comprises a helmet.

11. A system (1, 47) according to any of Claims 5 to 7 when dependent on Claim 4, wherein said display comprises a monitor with a resolution of at least 1280 x 1024 pixels.

12. A system (1, 47) according to Claim 11, wherein said line of sight sensor comprises an image capture device for capturing images of a marker that is provided in a known spatial relationship with an eye of a subject being tested.

13. A system (1, 47) according to any preceding claim, wherein the control module (5, 51) is capable of providing a plurality of different tests to the subject via the same test presentation device.

14. A system (1, 47) according to Claim 13, comprising a results analysis module (37) for considering results from a plurality of different tests undertaken by a subject.

15. A system (1, 47) according to Claim 14, wherein said control module (5, 51) is configured to be capable of adapting a selected test in accordance with the results for that test to provide an adapted test for storage.

16. A system (1, 47) according to Claim 15, wherein said adapted test is tailored in accordance with the results for that test to subject problem areas of a subject's eye to greater scrutiny than that provided by said selected test.

## Patentansprüche

1. Ein optometrisches Testsystem (1, 47), das Folgendes aufweist:
eine Testpräsentationseinrichtung (7, 53) zur Vorlage optischer Tests für ein Subjekt;
einen Datenspeicher (3, 49) für eine Bibliothek optometrischer Tests, wobei jeder dieser Tests Anweisungen zur Bereitstellung optischer Stimuli an ein Subjekt aufweist;
ein Steuermodul (5, 51), das auf die im Datenspeicher (3, 49) für einen ausgewählten Test gespeicherten Anweisungen reagiert, um die Testpräsentationseinrichtung (7, 53) zu steuern und die Stimuli für den betreffenden Test an das Subjekt bereitzustellen, und
eine Eingabeschnittstelle (9, 55), die durch das Subjekt betreibbar ist, um Signale als Reaktion auf die Stimuli eines ausgewählten Tests zu erzeugen, die dem betreffenden Subjekt präsentiert werden, wobei das Steuermodul (5, 51) konfiguriert ist, um die Signale in Abhängigkeit von den Stimuli des ausgewählten Tests von der Eingabeschnittstelle (9, 55) zu empfangen;
wobei das optometrische Testsystem (1, 47) weiter ein Sensormodul (13, 57) aufweist, wobei das Steuermodul (5, 51) konfiguriert ist, um die Testpräsentationseinrichtung (7, 53) entsprechend vom Sensormodul (13, 51) empfangenen Signalen zu steuern, **dadurch gekennzeichnet, dass** das Sensormodul (13, 57) eine Reihe Sichtsensoren aufweist und dass das Steuermodul (5, 51) konfiguriert ist, um eine Teststrategie eines ausgewählten Tests als Reaktion auf Signale, die von der Reihe Sichtsensoren empfangen wurden, anzupassen.

2. System (1, 47) nach Anspruch 1, wobei das Steuermodul (5, 51) konfiguriert ist, um eine Teststrategie eines ausgewählten Tests anzupassen, indem Anzeigestellen für Stimuli variiert werden.

3. System (1, 47) nach Anspruch 1, wobei die Reihe der Sichtsensoren und das Steuermodul (5, 51) zusammenwirken, um die Bewegung eines Auges eines Subjekts zu erfassen.

4. System (1, 47) nach Anspruch 1, wobei die Reihe der Sichtsensoren und das Steuermodul (5, 51) zusammenwirken, um die Bewegung des Kopfs eines Subjekts zu erfassen.

5. System (1, 47) nach einem der Ansprüche von 1 bis 4, wobei das Sensormodul (13, 57) einen oder mehrere Umgebungssensor(en) aufweist.

6. System (1, 47) nach Anspruch 1, wobei das Sensormodul (13, 57) einen Lichtintensitätssensor aufweist.

7. System (1, 47) nach einem der vorstehenden Ansprüche, wobei die Testpräsentationseinrichtung (7, 53) eine Anzeige aufweist.

8. System (1, 47) nach einem der Ansprüche von 5 bis 7, sofern diese von einem Ansprüche von 1 bis 3 abhängig sind, wobei die Anzeige eine Mikroanzeige aufweist und das System weiter eine Optik (11) aufweist, um auf der Anzeige angezeigte Bilder auf die Retina eines getesteten Subjekts zu projizieren.

9. System (1, 47) nach Anspruch 8, wobei mindestens die Anzeige und die Optik (11) als Komponenten einer am Kopf anbringbaren Einrichtung bereitgestellt sind.

10. System (1, 47) nach Anspruch 9, wobei die am Kopf anbringbare Einrichtung einen Helm aufweist.

11. System (1, 47) nach einem der Ansprüche von 5 bis 7, sofern diese von Anspruch 4 abhängig sind, wobei die Anzeige einen Monitor mit einer Auflösung von mindestens 1280 x 1024 Pixeln aufweist.

12. System (1, 47) nach Anspruch 11, wobei die Reihe Sichtsensoren eine Bilderfassungseinrichtung zur Erfassung von Bildern eines Markers aufweist, der in einer bekannten räumlichen Beziehung zu einem Auge des getesteten Subjekts bereitgestellt ist.

13. System (1, 47) nach einem der vorstehenden Ansprüche, wobei das Steuermodul (5, 51) eine Vielzahl unterschiedlicher Tests für das Subjekt über die gleiche Testpräsentationseinrichtung bereitstellen kann.

14. System (1, 47) nach Anspruch 13, das ein Ergebnisanalysemodul (37) aufweist, um Resultate einer Vielzahl unterschiedlicher Tests, die durch ein Subjekt vorgenommen wurden, zu berücksichtigen.

15. System (1, 47) nach Anspruch 14, wobei das Steuermodul (5, 51) konfiguriert ist, sodass es einen ausgewählten Test entsprechend den Resultaten für den betreffenden Test anpassen kann, um einen angepassten Test zur Speicherung bereitzustellen.

16. System (1, 47) nach Anspruch 15, wobei der angepasste Test entsprechend den Ergebnissen des betreffenden Tests an die subjektspezifischen Problembereiche des Auges eines Subjekts individuell mit größerer Genauigkeit angepasst ist als bei dem ausgewählten Test.

## Revendications

1. Système de test optométrique (1, 47) comprenant :
un dispositif de présentation de tests (7, 53) pour présenter des tests optiques à un sujet ;
un dispositif de stockage de données (3, 49) pour une bibliothèque de différents tests optométriques, chaque test précité comprenant des instructions pour fournir des stimuli optiques à un sujet ;
un module de commande (5, 51) répondant aux instructions stockées dans ledit dispositif de stockage de données (3, 49) pour un test sélectionné afin de commander le dispositif de présentation de tests (7, 53) pour fournir les stimuli pour ce test au sujet ; et
une interface d'entrée (9, 55) actionnable par le sujet pour générer des signaux en réponse aux stimuli d'un test sélectionné qui sont présentés audit sujet ; ledit module de commande (5, 51) étant configuré pour recevoir lesdits signaux provenant de ladite interface d'entrée (9, 55) en réponse aux stimuli dudit test sélectionné ;
dans lequel ledit système de test optométrique (1, 47) comprend en outre un module de capteur (13, 57), ledit module de commande (5, 51) étant configuré pour commander ledit dispositif de présentation de tests (7, 53) en fonction de signaux reçus provenant dudit module de capteur (13, 51), **caractérisé par le fait que** ledit module de capteur (13, 57) comprend un capteur de ligne de vision et **par le fait que** ledit module de commande (5, 51) est configuré pour ajuster une stratégie de test d'un test sélectionné en réponse à des signaux reçus à partir dudit capteur de ligne de vision.

2. Système (1, 47) selon la revendication 1, dans lequel ledit module de commande (5, 51) est configuré pour ajuster une stratégie de test d'un test sélectionné par variation de positions d'affichage pour des stimuli.

3. Système (1, 47) selon la revendication 1, dans lequel ledit capteur de ligne de vision et ledit module de commande (5, 51) coopèrent pour suivre le mouvement d'un oeil d'un sujet.

4. Système (1, 47) selon la revendication 1, dans lequel ledit capteur de ligne de vision et ledit module de commande (5, 51) coopèrent pour suivre le mouvement de la tête d'un sujet.

5. Système (1, 47) selon l'une quelconque des revendications 1 à 4, dans lequel ledit module de capteur (13, 57) comprend un ou plusieurs capteurs environnementaux.

6. Système (1, 47) selon la revendication 5, dans lequel ledit module de capteur (13, 57) comprend un capteur d'intensité lumineuse.

7. Système (1, 47) selon l'une quelconque des revendications précédentes, dans lequel ledit dispositif de présentation de tests (7, 53) comprend un dispositif d'affichage.

8. Système (1, 47) selon l'une quelconque des revendications 5 à 7 lorsqu'elles sont dépendantes de l'une quelconque des revendications 1 à 3, dans lequel ledit dispositif d'affichage comprend un micro-affichage et ledit système comprend en outre une optique (11) pour projeter des images affichées sur ledit dispositif d'affichage sur la rétine d'un sujet qui est testé.

9. Système (1, 47) selon la revendication 8, dans lequel au moins ledit dispositif d'affichage et ladite optique (11) sont fournis en tant que composants d'un dispositif pouvant être monté sur tête.

10. Système (1, 47) selon la revendication 9, dans lequel ledit dispositif pouvant être monté sur tête comprend un casque.

11. Système (1, 47) selon l'une quelconque des revendications 5 à 7 lorsqu'elles sont dépendantes de la revendication 4, dans lequel ledit dispositif d'affichage comprend un écran ayant une résolution d'au moins 1280 x 1024 pixels.

12. Système (1, 47) selon la revendication 11, dans lequel ledit capteur de ligne de vision comprend un dispositif de capture d'image pour capturer des images d'un marqueur qui est disposé selon une relation spatiale connue avec un oeil d'un sujet qui est testé.

13. Système (1, 47) selon l'une quelconque des revendications précédentes, dans lequel le module de commande (5, 51) est apte à fournir une pluralité de tests différents au sujet par l'intermédiaire du même dispositif de présentation de tests.

14. Système (1, 47) selon la revendication 13, comprenant un module d'analyse de résultats (37) pour prendre en compte des résultats provenant d'une pluralité de tests différents entrepris par un sujet.

15. Système (1, 47) selon la revendication 14, dans lequel ledit module de commande (5, 51) est configuré pour être apte à adapter un test sélectionné selon les résultats de ce test afin de fournir un test adapté pour stockage.

16. Système (1, 47) selon la revendication 15, dans lequel ledit test adapté est ajusté selon les résultats de ce test pour des zones problématiques de sujet d'un oeil du sujet pour un examen plus approfondi que celui fourni par ledit test sélectionné.
